# EUROPEAN PATENT SPECIFICATION

(11) **EP 1 411 887 B1**
(45) Date of publication and mention of the grant of the patent: **22.04.2009**
(21) Application number: 02758359.0
(22) Date of filing: 18.07.2002
(51) Int. Cl.: A61Q 17/04, A61K 8/11, A61K 8/35, A61K 8/37, A61K 8/40, A61K 8/64

(54) **SUNSCREEN COMPOSITION**
SONNENSCHUTZZUSAMMENSETZUNG
COMPOSITION ECRAN SOLAIRE

(30) Priority: 31.07.2001 EP 01117748
(43) Date of publication of application: 28.04.2004
(73) Proprietor: Merck Patent GmbH, 64293 Darmstadt (DE)
(72) Inventor: HARIVEL, Anne-Cécile, F-94370 Sucy-en-Brie (FR)
(86) International application number: PCT/EP2002/008010
(87) International publication number: WO 2003/011238

(56) References cited:
- EP-A- 0 193 932
- EP-A- 0 329 032
- EP-A- 1 216 682
- WO-A-00/78629
- WO-A-01/05366
- DE-A- 19 955 375
- FR-A- 2 209 561
- FR-A- 2 765 104
- US-A- 5 849 274
- DATABASE WPI Week 199421 Derwent Publications Ltd., London, GB; AN 1994-173640 XP002230541 & JP 06 116129 A (PIAS KK ), 26 April 1994 (1994-04-26)

## Description

The present invention relates to sunscreen compositions.

A suntan of the skin to whatever degree is regarded in today's society as attractive and as an expression of vigour and sportiness. As well as this desired effect of the sun on the skin, a number of undesired secondary effects arise, such as sunburn or premature skin ageing and the development of wrinkles. In the meantime, a number of performance UV filters have been developed which, applied to the skin in the form of creams, lotions or gels, can effectively delay the development of sunburn even when the incidence of solar rays is relatively high. The UV filter present in the pharmaceutical or cosmetic composition forms a film or a layer on the surface of the skin and does not penetrate into deeper skin layers with other care substances present in the composition. Known UV filters or sun protection agents thus act only by absorbing certain regions of sunlight, meaning that this radiation cannot penetrate into deeper layers of the skin. As is known, the most hazardous part of solar radiation is formed by the ultraviolet rays having a wavelength of less than 400 nm. The lower limit of the ultraviolet rays which reach the surface of the earth is limited by the absorption in the ozone layer to about 280 nm. The sun protection filters which are nowadays customary in cosmetics absorb in a wavelength range from 280 to 400 nm. This range includes UV-B rays having a wavelength between 280 and 320 nm, which play a decisive role in the formation of a solar erythema, and also UV-A rays, having a wavelength between 320 and 400 nm, which tan the skin but also age it, favour the triggering of an erythematous reaction or can exacerbate this reaction in certain people or even trigger phototoxic or photoallergic and irritative reactions.

The object of care cosmetics is wherever possible to obtain the impression of a youthful skin. In principle, there are various ways of achieving this object. For example, existing skin damage, such as irregular pigmentation or the development of wrinkles can be smoothed out by covering powders or creams. Another approach is to protect the skin against environmental influences which lead to permanent damage and thus ageing of the skin. The idea is therefore to intervene in a preventative manner and thus to delay the ageing process. One example of this is the UV filters already mentioned which, as a result of absorption of certain wavelength regions, prevent or at least reduce skin damage. Depending on the position of their absorption maxima, UV absorbers for cosmetic and dermatological compositions are divided into UV-A and UV-B absorbers. UV-A absorbers are usually also absorbing in the UV-B region and thus alternatively also being referred to as broad-band absorbers or broad-band filters.

Of decisive importance for the formulation is the solubility of the filter substances in the oil and water phases since it is necessary, particularly for establishing a high protection factor, to incorporate filters into all phases of the formulation. The oil-soluble UV-B filters include isooctyl methoxycinnamate, isoamyl methoxycinnamate and methylbenzylidene-camphor. Examples of water-soluble UV filters are, in particular, the salts of 2-phenylbenzimidazole-5-sulfonic acid, the use of which as UV ray filter has already been described in German Reichspatent No. 676 103.

In DE-A-199 55 375 a foamable sunscreen formulation being a oil-in-water-emulsion in a foam dispenser is described. The emulsion contains 2-50 % by weight of a oil phase and 50 to 98 % by weight of a water phase, wherein the water phase contains a surfactant and the emulsion contains 1-40 % by weight of UV filters being divided between the oil and water phase in a manner that the water phase contains 0 to 10 % by weight of the filters and the oil phase contains 1 to 30 % by weight of the filters.

In the European patent application EP-A-0 193 932 a sunscreen composition for hair protection is disclosed. The composition comprises a mousse base or concentrate containing a sunscreen agent therein, said mousse base or concentrate comprising a cationic surfactant substantive to hair and a non-ionic film former which in combination with non-ionic surfactant produces foam in the composition and upon application to hair forms a coating thereon. The composition is limited to water soluble or at least water miscible sunscreen agents.

JP 06-116129 discloses microcapsules containing benzophenone-type sunscreen agents in a foamable composition.

There was therefore a need for a foamable sunscreen composition with high sun protection factor (SPF) without giving an oily feeling when applied to the skin.

It has now surprisingly been found that this can be acheived with a aqueous composition comprising hydrophobic organic sunscreen agents and foam builder / stabilisers according to claim 1.

The present invention thus firstly provides a foamable aqueous composition having UV protection properties comprising at least one hydrophobic organic sunscreen agent and at least one foam builder / stabiliser according to claim 1.

Advantages of the compositions according to our invention are:
- High Sun protection factor (SPF).
- Good photostability of the composition.
- Hydrophobic UV filters are present in a purely aqueous formulation.
- The oily impression which is often perceived as unpleasant upon application of the preparation comprising hydrophobic UV filters is prevented.
- Good applicability due to stable foam.

The foamable composition according to our invention is able to be foamed up with or without an propellant. According to our invention it is especially preferred if the foam is produced without the use of a organic propellant. Sprays using organic propellents may not be stored in direct sun or at higher temperatures; conditions that for example can often be found on the beach during summer. An advantage of preferred compositions according to our invention is that the may be stored and used even under these conditions.

Therefore an embodiment of our invention is a kit of parts comprising a foam dispenser and a composition according to our invention. Suitable foam dispensers are known to those skilled in the art. A preferred dispenser for this kit-of-parts is described in WO 00/78629.

The dispenser of WO 00/78629 is suitable for dispensing a liquid, in particular in the form of a foam, and comprises at least a liquid container and a dispensing assembly which is coupled thereto at least in liquid-tight manner. A preferred dispensing assembly comprises a liquid pump with a liquid inlet and a liquid outlet and an actuating head. The actuating head comprises an outlet passage and a dispensing opening for dispensing the liquid, while the actuating head furthermore comprises a closed, circumferential protective cap. The aerosol furthermore comprises a circumferential recess, into which the protective cap can move, which recess comprises a closed inner wall, an outer wall and a base. Preferrably one or more outlet openings are present in the vicinity of the base of the recess, in which aerosol the shape of the recess is such that the inner wall, on the side which is remote from the base, is at a radial distance p from the protective cap, which distance p is greater than the usual clearance distance of the protective cap with respect to the inner wall.

Preferred compositions are included in a foam dispenser, preferably in a foam dispenser that requires no organic propellant as described above.

As foam builders / stabilisers according to our invention anionic protein derivatives may be used. Those substances in general are known to those skilled in the art. Preferred foam builders / stabilisers are those which are skin tolerant or even more preferrably give a benefit to the skin.

The foam builders / stabilisers are usually present in an amount of about 0.01 to 20 % by weight, preferably in an amount of 0.1 to 5 % by weight and even more preferred in an amount of 0.1 to 3 % by weight.

In a preferred embodiment the foam stabilisers according to our invention are so called foam boosters. Foam boosters are substances which increase the surface viscosity of the liquid which surrounds the individual bubbles in a foam. These agents are commonly used in shaving soaps, shampoos, bubble baths, liquid soaps, mousses, or aerosol-dispensed foams. Also *Film Formers* or *Viscosity-Increasing Agents* maybe used as foam boosters. The listing below gives examples for foam boosters which can also be classified as surfactants (INCI names):
Acetamide MEA, Almondamide DEA, Almondamidopropylamine Oxide, Almondamidopropyl Betaine, Apricotamide DEA, Apricotamidopropyl Betaine, Avocadamide DEA, Avocadamidopropyl Betaine, Babassuamide DEA, Babassuamidopropylamine Oxide, Babassuamidopropyl Betaine, Behenamide DEA, Behenamide MEA, Behenamidopropyl Betaine, Behenamine Oxide, Behenyl Betaine, Canolamidopropyl Betaine, Capramide DEA, Carnitine, Cetearyl Alcohol, Cetyl Alcohol, Cetyl Betaine, Cocamide DEA, Cocamide MEA, Cocamide MIPA, Cocamidoethyl Betaine, Cocamidopropylamine Oxide, Cocamidopropyl Betaine, Cocamidopropyl Hydroxysultaine, Cocamine Oxide, Cocoamphodipropionic Acid, Cocobetainamido Amphopropionate, Coco-Betaine, Coco-Hydroxysultaine, Coco-Morpholine Oxide, Coconut Alcohol, Coco/Oleamidopropyl Betaine, Coco-Sultaine, Cocoyl Sarcosinamide DEA, DEA-Cocoamphodipropionate, DEA-Lauraminopropionate, Decyl Alcohol, Decylamine Oxide, Decyl Betaine, Diethanolaminooleamide DEA, Dihydroxyethyl C8-10 Alkoxypropylamine Oxide, Dihydroxyethyl C9-11 Alkoxypropylamine Oxide, Dihydroxyethyl C12-15 Alkoxypropylaminde Oxide, Dihydroxyethyl Cocamine Oxide, Dihydroxyethyl Lauramine Oxide, Dihydroxyethyl Stearamine Oxide, Dihydroxyethyl Tallowamine Oxide, Dimethicone Propyl PG-Betaine, Disodium Caproamphodiacetate, Disodium Caproamphodipropiante, Disodium Capryloamphodiacetate, Disodium Capryloamphodipropionate, Disodium Cetearyl SulfosuccinateDisodium Cocamido MIPA-Sulfosuccinate, Disodium Cocamido PEG-3 Sulfosuccinate, Disodium Cocaminopropyl Iminodiacetate, DisodiumCocoamphocarboxyethylhydroxypropylsulfonate, Disodium Cocoamphodiacetate, Disodium Cocoamphodipropionate, Disodium C12-15 Pareth Sulfosuccinate, Disodium Deceth-5 Sulfosuccinate, Disodium Deceth-6 Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearoamphodiacetate, Disodium Isostearoamphodipropionate, Disodium Isostearyl Sulfosuccinate, Disodium Laneth-5 Sulfosuccinate, Disodium Lauramido MEA-Sulfosuccinate, Disodium Lauramido PEG-2 Sulfosuccinate, Disodium Laureth-5 Carboxyamphodiacetate, Disodium Laureth Sulfosuccinate, Disodium Laureth-6 Sulfosuccinate, Disodium Laureth-9 Sulfosuccinate, Disodium Laureth-12 Sulfosuccinate, Disodium Lauroamphodiacetate, Disodium Lauroamphodipropiante, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleoamphodipropionate, Disodium Oleth-3 Sulfosuccinate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PPG-2-Isodeceth-7 Carboxyamphodiacetate, Disodium Ricinoleamido MEA-Sulfosuccinate, Disodium Stearamido MEA-Sulfosuccinate, Disodium Stearoamphodiacetate, Disodium Stearyl Sulfosuccinamate, Disodium Stearyl Sulfosuccinate, Disodium Tallamido MEA-Sulfosuccinate, Disodium Tallowamido MEA-Sulfosuccinate, Disodium Tallowamphodiacetate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Wheat Germamido MEA-Sulfosuccinate, Disodium Wheat Germamido PEG-2 Sulfosuccinate, Disodium Wheatgermamphodiacetate, Di-TEA-Oleamido PEG-2 Sulfosuccinate, Ditridecyl Sodium Sulfosuccinate, Erucamidopropyl Hydroxysultaine, Hydrogenated Tallow Alcohol, Hydrogenated Tallowamide DEA, Hydrogenated Tallowamine Oxide, Hydrogenated Tallow Betaine, Hydroxyethyl Carboxymethyl Cocamidopropylamine, Hydroxyethly Hydroxypropyl C12-15 Alkoxypropylamine Oxide, Hydroxystearamide MEA, Isostearamide DEA, Isostearamide MEA, Isostearamide MIPA, Isostearamidopropylamine Oxide, Isostearamidopropyl Betaine, Isostearamidopropyl Morpholine Oxide, Lactamide MEA, Lanolinamide DEA, Lauramide DEA, Lauramide MEA, Lauramide MIPA, Lauramide/Myristamide DEA, Lauramidopropylamine Oxide, Lauramidopropyl Betaine, Lauramine Oxide, Lauroamphodipropionic Acid, Lauryl Alcohol, Lauryl Betaine, Lauryl Hydroxysultaine, Lauryl Sultaine, Lecithinamide DEA, Linoleamide DEA, Linoleamide MEA, Linoleamide MIPA, Methyl Morpholine Oxide, Minkamide DEA, Minkamidopropylamine Oxide, Minkamidopropyl Betaine, Myristamide DEA, Myristamide MEA, Myristamide MIPA, Myristamidopropylamine Oxide, Myristamidopropyl Betaine, Myristamine Oxide, Myristaminopropionic Acid, Myristyl Alcohol, Myristyl Betaine, Myristyl/Cetyl Amine Oxide, Oleamide DEA, Oleamide MEA, Oleamide MIPA, Oleamidopropylamine Oxide, Oleamidopropyl Betaine, Oleamidopropyl Hydroxysultaine, Oleamine Oxide, Oleyl Betaine, Olivamide DEA, Olivamidopropylamine Oxide, Olivamidopropyl Betaine, Palmamide DEA, Palmamide MEA, Palmamide MIPA, Palmamidopropyl Betaine, Palmitamide DEA, Palmitamide MEA, Palmitamidopropylamine Oxide, Palmitamidopropyl Betaine, Palmitamine Oxide, Palm Kernel Alcohol, Palm Kernelamide DEA, Palm Kernelamide MEA, Palm Kernelamide MIPA, Palm Kernelamidopropyl Betaine, Peanutamide MEA, Peanutamide MIPA, PEG-3 Cocamide, PEG-2 Hydrogenated Tallow Amine, PEG-3 Lauramide, PEG-3 Lauramide Oxide, PEG-2 Oleamide, PEG-3 Oleamide, PEG-2 Oleamine, PEG-2 Soyamine, PEG-2 Stearamine, Potassium Dihydroxyethyl Cocamine Oxide Phosphate, Ricinoleamide DEA, Ricinoleamide MEA, Ricinoleamide MIPA, Ricinoleamidopropyl Betaine, Sesamide DEA, Sesamidopropylamine Oxide, Sesamidopropyl Betaine, Sodium Caproamphoacetate, Sodium Caproamphohydroxypropylsulfonate, Sodium Caproamphopropionate, Sodium Capryloamphoacetate, Sodium Capryloamphohydroxypropylsulfonate, Sodium Capryloamphoproprionate, Sodium Cocoamphoacetate, Sodium Cocoamphohydroxypropylsulfonate, Sodium Cocoamphopropionate, Sodium Cornamphopropionate, Sodium Isostearoamphoacetate, Sodium Isostearoamphopropionate, Sodium Lauramidopropyl Hydroxyphostaine, Sodium Lauraminopropionate, Sodium Lauriminodipropionate, Sodium Lauroamphoacetate, Sodium/MEA Laureth-2 Sulfosuccinate, Sodium Myristoamphoacetate, Sodium Oleoamphoacetate, Sodium Oleoamphohydroxypropylsulfonate, Sodium Oleoamphopropionate, Sodium Ricinoleoamphoacetate, Sodium Stearoamphoacetate, Sodium Stearoamphohydroxypropylsulfonate, Sodium Stearoamphopropionate, Sodium Tallamphopropionate, Sodium Tallowamphoacetate, Sodium Tallowate, Sodium Undecylenoamphoacetate, Sodium Undecylenoamphopropionate, Sodium Wheat Germamphoacetate, Soyamide DEA, Soyamidopropyl Betaine, Stearamide AMP, Stearamide DEA, Stearamide DEA-Distearate, Stearamide MEA, Stearamide MEA-Stearate, Stearamide MIPA, Stearamidopropylamine Oxide, Stearamidopropyl Betaine, Stearamine Oxide, Stearyl Alcohol, Stearyl Betaine, Tallamide DEA, Tallowamide DEA, Tallowamide MEA, Tallowamidopropylamine Oxide, Tallowamidopropyl Betaine, Tallowamidopropyl Hydroxysultaine, Tallowamine Oxide, Tallow Betaine, TEA-Lauraminopropionate, TEA-Myristaminopropionate, Trideceth-2 Carboxamide MEA, Trisodium Lauroampho PG-Acetate Phosphate Chloride, Undecylenamide DEA, Undecylenamide MEA, Undecylenamidopropylamine Oxide, Undecylenamidopropyl Betaine, Wheat Germamide DEA, Wheat Germamidopropylamine Oxide, Wheat Germamidopropyl Betaine.

In the present invention, at least one foam builder/stabiliser is selected from anionic protein derivatives, such as lipoaminoacids described in WO98/09611, WO 99/27902 and WO 99/45899. Most preferred under these anionic protein derivatives are sodium lauroyl oat amino acids, for example known as Proteol^{™} Oat (Tradename of Seppic).

According to claim 1, selected hydrophobic filters are included in encapsulated form. Particular preference is given to those UV filters whose physiological safety has already been demonstrated. Among the many tried and tested substances known from the specialist literature for both UVA and also UVB filters, there are the hydrophobic organic sunscreen agents according to claim 1, namely
- 3-(4'-methylbenzylidene)-dl-camphor (e.g. Eusolex^{®} 6300),
- 1-(4-tert-butylphenyl)-3-(4-methoxyphenyl)propane-1,3-dione (e.g. Eusolex^{®} 9020) or
- 4-isopropyldibenzoylmethane (e.g. Eusolex^{®} 8020),
- 2-hydroxy-4-methoxybenzophenone (e.g. Eusolex^{®} 4360)
- octyl methoxycinnamate (e.g. Eusolex^{®} 2292),
- 3,3,5-trimethylcyclohexyl salicylate (e.g. Eusolex^{®} HMS),
- 2-ethylhexyl 4-(dimethylamino)benzoate (e.g. Eusolex^{®} 6007), and/or
- 2-ethylhexyl 2-cyano-3,3-diphenylacrylate (e.g. Eusolex^{®} OCR),

These organic UV filters are usually incorporated into cosmetic formulations in an amount of from 0.5 to 20% by weight, preferably 1 - 15%.

By combining two or more compounds listed above it is possible to optimize the protective action against harmful effects of UV radiation. The combination of the above-mentioned UV filters in a formulation gives a composition which combines light protection with particular mildness to the skin. All of the UV filters specified are used in encapsulated form. In particular according to our invention it is advantageous to use hydrophobic UV filters in encapsulated form.

In a preferred embodiment of our invention, the composition further comprises at least one water-soluble organic sunscreen agent, preferably in an amount from 0.01 to 20 % by weight.

It is also preferred, if the composition further comprises at least one inorganic sunscreen agent, preferably at least one microparticulate inorganic sunscreen, most preferably selected from zink or titanium dioxide.

According to the invention one or more of the above-mentioned hydrophobic UV filters are present in encapsulated form. In this connection, it is advantageous if the capsules are so small that they can not be observed with the naked eye. To achieve the above-mentioned effects, it is also necessary for the capsules to be sufficiently stable and not to release the encapsulated active ingredient (UV filter) into the surroundings, or to release it only to a slight extent.

Suitable capsules can have walls made of inorganic or organic polymers. For example, US 6,242,099 B1 describes the preparation of suitable capsules with balls made of chitin, chitin derivatives or polyhydroxylated polyamines. Capsules which are to be used particularly preferably according to the invention have walls which can be obtained by a sol-gel process, as is described in the applications WO 00/09652, WO 00/72806 and WO 00/71084. Preference is given here in turn to capsules whose walls are made of silica gel (silica; undefined silicon oxide hydroxide). The preparation of corresponding capsules is known to the person skilled in the art, for example, from the cited patent applications, the contents of which also expressly belonging to the subject-matter of the present application.

Preferred capsules have a average particle size in the range from about 10 nm up to about 10000 nm, preferably up to 5000 nm and most preferred up to 2000 nm. The minimal particle size of those capsules depends on necessary size to prevent penetration of the skin. On the other side, the maximum particle size is limited by the application needs. The capsules shouldn't be discernible with blank eyes.

Here, the capsules are present in formulations according to the invention preferably in amounts which ensure that the encapsulated UV filters are present in the formulation in the amounts given above.

In a preferred embodiment of our invention the hydrophobic sunscreen is preferably encapsulated in capsules mainly consisting of organic polymeric materials or inorganic oxidic materials as described above.

The protecting action against oxidative stress or against the effect of free radicals can be further improved if the formulation comprises one or more antioxidants.

There are many tried and tested substances known from the specialist literature which can be used, e.g. amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotinoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glycerylesters thereof), and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthionine-sulfoximine, homocysteine-sulfoximine, buthionine-sulfone, penta-, hexa- and heptathionine-sulfoximine) in very low tolerated doses (e.g. pmol to µmol/kg), and also (metal) chelating agents, (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof, vitamin C and derivatives (e.g. ascorbyl palmitate, magnesium ascorbyl phosphate, ascorbyl acetate), tocopherols and derivatives (e.g. vitamin E acetate), vitamin A and derivatives (e.g. vitamin A palmitate), and coniferyl benzoate of benzoin resin, rutin and salts of the sulfuric ester of rutin and derivatives thereof, α-glycosyl rutin, ferulic acid, furfurylidineglucitol, carosine, butylhydroxytoluene, butylhydroxyanisol, nordihydroguaretic acid, trihydroxybutyrophenone, quercetin, uric acid and derivatives thereof, mannose and derivatives thereof, zinc and derivatives thereof (e.g. ZnO, ZnSO₄), selenium and derivatives thereof (e.g. selenomethionine), stilbenes and derivatives thereof (e.g. stilbene oxide, trans-stilbene oxide).

Mixtures of antioxidants are likewise suitable for use in the cosmetic formulations according to the invention. Known and commercial mixtures are, for example, mixtures comprising, as active ingredients, lecithin, L-(+)-ascorbyl palmitate and citric acid (e.g. Oxynex^{®} AP), natural tocopherols, L-(+)-ascorbyl palmitate, L-(+)-ascorbic acid and citric acid (e.g. Oxynex^{®} K LIQUID), tocopherol extracts from natural sources, L-(+)-ascorbyl palmitate, L-(+)-ascorbic acid and citric acid (e.g. Oxynex^{®} L LIQUID), DL-α-tocopherol, L-(+)-ascorbyl palmitate, citric acid and lecithin (e.g. Oxynex^{®} LM) or butylhydroxytoluene (BHT), L-(+)-ascorbyl palmitate and citric acid (e.g. Oxynex^{®} 2004).

The formulations according to the invention can comprise vitamins as further ingredients. Preferably, vitamins and vitamin derivatives chosen from vitamin A, vitamin A propionate, vitamin A palmitate, vitamin A acetate, retinol, vitamin B, thiamine chloride hydrochloride (vitamin B₁), riboflavin (vitamin B₂) nicotinamide, vitamin C (ascorbic acid), vitamin D, ergocalciferol (vitamin D₂), vitamin E, DL-α-tocopherol, tocopherol E acetate, tocopherol hydrogensuccinate, vitamin K₁, esculin (vitamin P active ingredient), thiamine (vitamin B₁) nicotinic acid (niacin), pyridoxine, pyridoxal, pyridoaxmine, (vitamin B₆), panthothenic acid, biotin, folic acid and cobalamine (vitamin B₁₂) are present in the cosmetic formulations according to the invention, particularly preferably vitamin A palmitate, vitamin C, DL-α-tocopherol, tocopherol E acetate, nicotinic acid, panthothenic acid and biotin.

The composition of our invention is a cosmetic formulation.

Any customary carriers, auxiliaries and optionally further active ingredients may be added to the formulation. Preferred auxiliaries originate from the group of preservatives, antioxidants, stabilizers, solubility promoters, vitamins, colorants, odour improvers.

Further typically cosmetic application forms are also sunscreen, presun and aftersun compositions.

All compounds or components which can be used in the cosmetics formulations are either known and available commercially or can be synthesized by known processes.

The composition according to the invention is particularly suitable for protecting human skin against the harmful influences of the UV constituents in sunlight, in addition they also offer protection against ageing processes of the skin and against oxidative stress, i.e. against damage caused by free radicals, as are produced, for example, by solar irradiation, heat or other influences.

The formulation may comprise adjuvants which are customarily used in this type of composition, such as, for example, thickeners, softeners, moisturizers, surface-active agents, emulsifiers, preservatives, perfumes, waxes, lanolin, propellants, dyes and/or pigments which colour the composition itself or the skin, and other ingredients customarily used in cosmetics.

If a composition is formulated as an aerosol, the customary propellants, such as alkanes, fluoroalkanes and chlorofluoroalkanes may be used.

The cosmetic formulation can also be used to protect the hair against photochemical damage in order to prevent changes of colour shades, decoloration or damage of a mechanical nature. In this case, a suitable formulation is in the form of a shampoo or lotion for rinsing out, the formulation in question being applied before or after shampooing, before or after colouring or bleaching or before or after permanent waving. It is also possible to choose a formulation in the form of a lotion or gel for styling or treating the hair, in the form of a lotion or gel for brushing or blow-waving, in the form of a hair lacquer, permanent waving composition, colorant or bleach for the hair. The cosmetic formulation may comprise various adjuvants used in this type of composition, such as surface-active agents, thickeners, polymers, softeners, preservatives, foam stabilizers, electrolytes, organic solvents, silicone derivatives, antigrease agents, dyes and/or pigments which colour the composition itself or the hair, or other ingredients customarily used for hair care.

To protect the skin and/or natural or sensitized hair against solar rays, the cosmetic composition is applied to the skin or the hair. Sensitized hair is understood here as meaning hair which has been subjected to a chemical treatment, such as a permanent waving treatment, a colouring process or bleaching process.

The compositions of our invention can be produced by mixing aqueous dispersions of encapsulated organic sunscreens and/or aqueous dispersions of inorganic sunscreen particles with other ingredients of the composition. This method for producing a sunscreen is another preferred embodiment of our invention.

The examples below illustrate the present invention in more detail without limiting the scope of the invention. The following trade names are used in the example formulations:

The silica capsules used in the examples can be obtained by a sol-gel process, as is described in the applications WO 00/09652, WO 00/72806 and WO 00/71084. The preparation of corresponding capsules is known to the person skilled in the art, for example, from the cited patent applications.

### Example 1: SUN PROTECTION SPRAY-Mousse

| | supplier | % by weight |
|---|---|---|
| PHASE A | | |
| Water, Titanium dioxide, Alumina, sodium metaphosphate, phenoxyethanol, sodium methyl paraben (Eusolex^{™} T aqua) | 1 | 16.5 |

| PHASE B | | |
|---|---|---|
| Phenyl benzimidazole Sulfonic Acid (Eusolex^{™} 232) | 1 | 3 |
| Sodium hydroxyde | 1 | 0.44 |
| Water | | 10 |

| PHASE C | | |
|---|---|---|
| CI 77891 (Titanium dioxide), Mica, Silica (Timiron^{™} Splendid gold) | 1 | 1 |
| Sodium Lauroyl OAT Aminoacids (Proteol^{™} oat) | 2 | 5 |
| Dimethicone Copolyol Phosphate (Pecosil^{™} PS 100) | 2 | 0.5 |
| Disodium EDTA | 1 | 0.1 |
| Chlorphenesin | 1 | 0.3 |
| Glycerol | 1 | 3 |
| Water, demineralized | | Qsp 100 |

| PHASE D | | |
|---|---|---|
| silica capsules OMC (Octylmethoxycinnamate: 42%) | 1 | 23 |

### PROCEDURE:

Phase A is dispersed in the Phase C. Then phase B and phase D are added and neutralized at pH=8.

| | | |
|---|---|---|
| Suppliers: | 1 | Merck |
| | 2 | Seppic |

### Example 2: SUN PROTECTION SPRAY-Mousse

| | supplier | % by weight |
|---|---|---|
| PHASE A | | |
| Water, Titanium dioxide, Alumina, sodium metaphosphate, phenoxyethanol, sodium methyl paraben (Eusolex^{™} T aqua) | 1 | 16 |

| PHASE B | | |
|---|---|---|
| Sodium Lauroyl OAT Aminoacids (protect oat) | 2 | 5 |
| Disodium EDTA | 1 | 0.1 |
| Chlorphenesin | 1 | 0.3 |
| Glycerol | 1 | 3 |
| Water, demineralized | | Qsp 100 |

| PHASE C | | |
|---|---|---|
| silica capsules OMC (Octylmethoxycinnamate: 42%) | 1 | 23 |

### PROCEDURE:

Phase B is dispersed in the Phase A. Then phase C is added and neutralized at pH=5,5.

| | | |
|---|---|---|
| Suppliers: | 1 | Merck |
| | 2 | Seppic |

### Example 3: SUN PROTECTION SPRAY-Mousse

| | supplier | % by weight |
|---|---|---|
| PHASE A | | |
| Water, Titanium dioxide, Alumina, sodium metaphosphate, phenoxyethanol, sodium methyl paraben (Eusolex^{™} T aqua) | 1 | 16.5 |

| PHASE B | | |
|---|---|---|
| Phenyl benzimidazole Sulfonic Acid (Eusolex^{™} 232) | 1 | 3 |
| Sodium hydroxyde | 1 | 0.44 |
| Water | | 10 |

| PHASE C | | |
|---|---|---|
| Sodium Lauroyl OAT Aminoacids (protect oat) | 2 | 5 |
| Disodium EDTA | 1 | 0.1 |
| Chlorphenesin | 1 | 0.3 |
| Glycerol | 1 | 3 |
| Water, demineralized | | Qsp 100 |

| PHASE D | | |
|---|---|---|
| silica capsules OMC (Octylmethoxycinnamate: 42%) | 1 | 23 |

### PROCEDURE:

Phase A is dispersed in the Phase C. Then phase B and phase D are added and neutralized at pH=8.

| | | |
|---|---|---|
| Suppliers: | 1 | Merck |
| | 2 | Seppic |

The average SPC (3 measurements), measured for a layer of 2 mg/cm² on Transpore^{™} Tape (trademark of 3M) 20 min after application, is 22,4.

### Example 4:

| | |
|---|---|
| Eusolex^{™} T aqua | 16.5 |
| Eusolex^{™} 232 | 3 |
| Eusolex^{™} 9020 in silica capsule | 23 |
| Sodium hydroxyde | 0.44 |
| Proteol^{™} oat | 5 |
| Disodium EDTA | 0.1 |
| Chlorphenesin1 | 0.3 |
| Glycerol | 3 |
| Water, demineralized | Qsp 100 |

### Example 5:

| | |
|---|---|
| Eusolex^{™} T aqua | 16.5 |
| Eusolex^{™} 232 | 3 |
| Eusolex^{™} 6300 in silica capsule | 20 |
| Sodium hydroxyde | 0.44 |
| Proteol^{™} oat | 5 |
| Disodium EDTA | 0.1 |
| Chlorphenesin1 | 0.3 |
| Glycerol | 3 |
| Water, demineralized | Qsp 100 |

### Example 6:

| | |
|---|---|
| Eusolex^{™} T aqua | 16.5 |
| Eusolex^{™} 232 | 3 |
| Eusolex^{™} OCR in silica capsule | 12 |
| Eusolex^{™} 9020 in silica capsule | 12 |
| Sodium hydroxyde | 0.44 |
| Proteol^{™} oat | 5 |
| Disodium EDTA | 0.1 |
| Chlorphenesin1 | 0.3 |
| Glycerol | 3 |
| Water, demineralized | Qsp 100 |

### Example 7:

| | |
|---|---|
| Eusolex^{™} T aqua | 16.5 |
| Eusolex^{™} 232 | 3 |
| Eusolex^{™} 9020/OCR in silica capsule | 12 |
| Eusolex^{™} 6300 in silica capsule | 12 |
| Sodium hydroxyde | 0.44 |
| Proteol^{™} oat | 5 |
| Disodium EDTA | 0.1 |
| Chlorphenesin1 | 0.3 |
| Glycerol | 3 |
| Water, demineralized | Qsp 100 |

### Example 8:

| | |
|---|---|
| Eusolex^{™} T-2000 | 12 |
| Eusolex^{™} 232 | 3 |
| Eusolex^{™} 9020 in silica capsule | 23 |
| Sodium hydroxyde | 0.44 |
| Proteol^{™} oat | 5 |
| Disodium EDTA | 0.1 |
| Chlorphenesin1 | 0.3 |
| Glycerol | 3 |
| Water, demineralized | Qsp 100 |

## Claims

1. Foamable aqueous cosmetic composition comprising at least one hydrophobic organic sunscreen agent selected from the group 3-(4'-methyl)-benzylidene)-dl-camphor, 1-(4 tert-butylphenyl)-3-(4--methoxyphenyl)-propane-1,3-dione, 4-isopropyldibenzoylmethane, 2-hydroxy-4-methoxybenzophenone, octyl methoxycinnamate, 3,3,5-trimethylcyclohexylsalicylate, 2-ethylhexyl 4-(dimethylamino)benzoate, 2-ethylhexyl 2-cyano-3,3-diphenylacrylate used in encapsulated form and at least one foam builder/stabiliser selected from anionic protein derivatives.

2. Composition according to Claim 1, **characterised in that** the composition comprises at least one water-soluble organic sunscreen agent, preferably in an amount from 0.01 to 20 % by weight.

3. Composition accordint to at least one of the claims 1 to 2, **characterised in that**, the composition comprises at least one inorganic sunscreen agent, preferably at least one microparticulate inorganic sunscreen, most preferably selected from zink or titanium dioxide.

4. Composition according to at least one of the claims 1 to 3, wherein the hydrophobic organic sunscreen agent is encapsulated in capsules mainly consisting of organic polymeric materials and/or inorganic oxidic materials.

5. Composition according to at least one of the claims 1 to 4, wherein anionic protein derivatives are sodium lauroyl OAT amino acids.

6. Composition according to at least one of the claims 1 to 5, **characterised in that** the composition is included in a foam dispenser, preferably in a foam dispenser that requires no propellant gas.

7. Kit of parts comprising a foam dispenser and a composition according to at least one of the claims 1 to 6.

8. Method for producing a composition according to at least one of the claims 1 to 6 by mixing aqueous dispersions of encapsulated sunscreens and/or aqueous dispersions of inorganic sunscreen particles with other ingredients of the composition.

## Patentansprüche

1. Schäumbare wässrige kosmetische Zusammensetzung enthaltend mindestens ein in verkapselter Form verwendetes, hydrophobes organisches Sonnenschutzmittel, ausgewählt aus der Gruppe 3-(4'-Methyl)-benzyliden)-dl-campher, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, Salicylsäure-3,3,5-trimethylcyclohexylester, 4-(Dimethylamino)benzoesäure-2-ethylhexylester, 2-Cyano-3,3-diphenylacrylsäure-2-ethylhexylester und mindestens einen Schaumbildner/-stabilisator ausgewählt aus anionischen Proteinderivaten.

2. Zusammensetzung nach Anspruch 1, **dadurch gekennzeichnet, dass** die Zusammensetzung mindestens ein wasserlösliches organisches Sonnenschutzmittel, vorzugsweise in einer Menge von 0,01 bis 20 Gew.-%, enthält.

3. Zusammensetzung nach mindestens einem der Ansprüche 1 bis 2, **dadurch gekennzeichnet, dass** die Zusammensetzung mindestens ein anorganisches Sonnenschutzmittel, vorzugsweise mindestens ein mikropartikuläres anorganisches Sonnenschutzmittel, insbesondere bevorzugt ausgewählt aus Zink oder Titandioxid, enthält.

4. Zusammensetzung nach mindestens einem der Ansprüche 1 bis 3, worin das hydrophobe organische Sonnenschutzmittel in Kapseln verkapselt ist, die überwiegend aus organischen polymeren Materialien und/oder anorganischen oxidischen Materialien bestehen.

5. Zusammensetzung nach mindestens einem der Ansprüche 1 bis 4, worin anionische Proteinderivate Natrium-Lauroyl-Hafer-Aminosäuren sind.

6. Zusammensetzung nach mindestens einem der Ansprüche 1 bis 5, **dadurch gekennzeichnet, dass** die Zusammensetzung in einen Schaumspender aufgenommen ist, vorzugsweise in einen Schaumspender, der kein Treibgas erfordert.

7. Set (Kit) enthaltend einen Schaumspender und eine Zusammensetzung nach mindestens einem der Ansprüche 1 bis 6.

8. Verfahren zur Herstellung einer Zusammensetzung nach mindestens einem der Ansprüche 1 bis 6, indem man wässrige Dispersionen verkapselter Sonnenschutzmittel und/oder wässrige Dispersionen anorganischer Sonnenschutzpartikel mit anderen Zutaten der Zusammensetzung mischt.

## Revendications

1. Composition cosmétique aqueuse expansible comprenant au moins un filtre solaire organique hydrophobe choisi parmi le groupe constitué par le 3-(4'-méthyl)benzylidène)-dl-camphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, utilisés sous forme encapsulée et au moins un stabilisant/adjuvant de mousse choisi parmi les dérivés protéiques anioniques.

2. Composition selon la revendication 1, **caractérisée en ce que** la composition comprend au moins un filtre solaire organique hydrosoluble, préférablement en une quantité allant de 0,01 à 20% en poids.

3. Composition selon au moins l'une des revendications 1 à 2, **caractérisée en ce que** la composition comprend au moins un filtre solaire inorganique, préférablement au moins un filtre solaire inorganique microparticulaire, tout préférablement choisi parmi le dioxyde de zinc ou de titane.

4. Composition selon au moins l'une des revendications 1 à 3, dans laquelle le filtre solaire organique hydrophobe est encapsulé dans des capsules constituées principalement de matériaux polymères organiques et/ou de matériaux d'oxyde inorganique.

5. Composition selon au moins l'une des revendications 1 à 4, dans laquelle les dérivés protéiques anioniques sont les sels de sodium d'acides aminés d'avoine modifiés par lauroyle.

6. Composition selon au moins l'une des revendications 1 à 5, **caractérisée en ce que** la composition est incluse dans un distributeur de mousse, préférablement dans un distributeur de mousse ne nécessitant aucun gaz propulseur.

7. Kit de pièces comprenant un distributeur de mousse et une composition selon au moins l'une des revendications 1 à 6.

8. Méthode de production d'une composition selon au moins l'une des revendications 1 à 6, par le mélange de dispersions aqueuses de filtres solaires encapsulés et/ou de dispersions aqueuses de particules de filtre solaire inorganique avec d'autres ingrédients de la composition.
